# DEMANDE DE BREVET EUROPEEN

(11) **EP 0 737 673 A1**
(43) Date de publication de la demande: **16.10.1996**
(21) Numéro de dépôt: 96400761.1
(22) Date de dépôt: 09.04.1996
(51) Int. Cl.: C07C 309/47, H01F 1/42, C09B 55/00

(54) **Composé moléculaire organique à propriétés ferromagnétiques et son procédé de fabrication**

(30) Priorité: 14.04.1995 FR 9504530
(71) Demandeur: ALCATEL ALSTHOM COMPAGNIE GENERALE D'ELECTRICITE, F-75008 Paris (FR)
(72) Inventeur: Galaj, Stanislas, 94110 Arcueil (FR); Le Mehaute, Alain, 91190 Gif sur Yvette (FR)
(74) Mandataire: Laroche, Danièle

(57) **Abrégé**

L'invention est un composé organique répondant à l'une des formules :

(I) L-A-Z

(II) L-A'-L'

dans lesquelles:
. -A- est un radical issu d'un noyau benzènique ou naphtalène,
. -Z est choisi parmi un radical hydro, nitrile, nitro, fluoro, chloro, bromo, iodo, amino, imino, alkylamino, dialkylamino, dialkyliminio, trialkylammonio, sulfo, carboxy, phosphono, arsono, acyle, formamido, acétamido, acétimido et aminocyclique,
. -A'- est un radical issu d'un noyau benzènique,
. -L et -L ' sont de la forme: 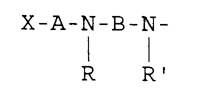 N étant l'atome d'azote, où:
. -X est choisi parmi un radical amino, imino, alkylamino, dialkylamino, dialkyliminio, trialkylammonio, sulfo, carboxy, phosphono, arsono et aminocyclique,
. -NR- et -NR'- sont des radicaux issus d'une amine primaire ou de l'ammoniac,
. -B- est un radical issu d'un noyau naphtalène ou benzènique portant au moins un substituant Y choisi parmi un radical sulfo, carboxy, phosphono, arsono, amino, immino, alkylamino, dialkylamino, dialkyliminio, trialkylammonio et aminocyclique.

## Description

La présente invention concerne une famille de composés moléculaires organiques à propriétés ferromagnétiques facilement cristallisables.

On a proposé un copolymère organique à propriétés ferromagnétiques dont le motif élémentaire comprend un premier groupe de composés amino-aromatiques, dérivés de la 1-naphtylamine et des formes iminoquinoniques correspondantes, accolé à un second groupe de composés aminoaromatiques substitués. Le second groupe est choisi parmi les composés aminés substitués comprenant au moins un motif aniline dans leur structure, les composés dérivant de l'aniline portant un substituant relié au noyau par un maillon éthynylène ou paraphénylène, et les composés iminoquinoniques correspondants. Ce copolymère est faiblement ferromagnétique et difficile à cristalliser.

Par ailleurs, on connaît des composés cristallins d'inclusion de sels métalliques dans ce copolymère, mais ceux-ci ne sont obtenus qu'avec des rendements relativement faibles.

La présente invention a pour but de proposer un composé moléculaire organique présentant une aimantation plus élevée que celles des copolymères connus.

L'invention a aussi pour but de proposer un composé dont la fabrication, et notamment la cristallisation, soit de réalisation facile et s'effectue avec des rendements améliorés.

La présente invention a pour objet un composé organique possédant des propriétés ferromagnétiques, qui est un composé moléculaire répondant à l'une des formules générales suivantes:

(I) L-A-Z

(II) L-A'-L'

dans lesquelles:
. -A- est choisi parmi un radical issu d'un noyau benzénique multivalent en position para et éventuellement substitué, un radical issu d'un noyau naphtalène multivalent en position para de l'un des noyaux et éventuellement substitué,
. -Z- est un radical mono ou bivalent choisi parmi un radical hydro, un radical nitrile, un radical nitro, un radical fluoro, un radical chloro, un radical bromo, un radical iodo, un radical amino, un radical imino, un radical alkylamino, un radical dialkylamino, un radical dialkyliminio, un radical trialkylammonio, un radical aminocyclique dont un atome de carbone du cycle peut être remplacé par un hétéroatome comme O, S ou N, un radical sulfo, un radical carboxy, un radical phosphono, un radical arsono, un radical acyle, un radical formamido, un radical acétamido et un radical acétimido.
. -A'- est choisi parmi un radical issu d'un noyau benzénique multivalent en position para ou ortho et éventuellement substitué,
. -L et -L' sont de la forme: 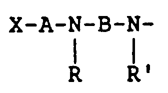 N étant l'atome d'azote, où:
. -X est un radical mono ou bivalent susceptible de fixer un proton, choisi parmi un premier groupe de composés constitué d'un radical amino, un radical imino, un radical alkylamino, un radical dialkylamino, un radical dialkyliminio, un radical trialkylammonio et un radical aminocyclique dont un atome de carbone du cycle peut être remplacé par un hétéroatome comme O, S ou N, et un deuxième groupe de composés constitué d'un radical sulfo, un radical carboxy, un radical phosphono et un radical arsono,
. -NR- et -NR'- sont des radicaux multivalents issus d'une amine primaire ou de l'ammoniac,
. -B- est choisi parmi un radical issu d'un noyau naphtalène multivalent en position para de l'un des noyaux et portant au moins un substituant Y en position 6 ou 7 sur l'autre noyau, un radical issu d'un noyau benzénique multivalent et portant au moins un substituant Y en position 2 ou 3 relié au noyau par un maillon éthynylène ou un maillon paraphénylène éventuellement substitué, ledit substituant Y étant choisi parmi un radical sulfo, un radical carboxy, un radical phosphono et un radical arsono si -X est choisi dans ledit premier groupe de composés, et ledit substituant Y étant choisi parmi un radical amino, un radical imino, un radical alkylamino, un radical dialkylamino, un radical dialkyliminio, un radical trialkylammonio et un radical aminocyclique dont un atome de carbone du cycle peut être remplacé par un hétéroatome comme O, S ou N, si -x est choisi dans ledit deuxième groupe de composés.

Le composé moléculaire organique selon la présente invention peut être décrit comme un enchaînement linéaire de radicaux multivalents. La nature des liaisons formant l'enchaînement dépend de l'état réduit ou oxydé de ces radicaux. Sous la forme réduite, -A-, -A'-, -B-, -NR- et -NR'- sont bivalents et -X, -Y et -Z sont monovalents. Sous la forme oxydée -A-, -A'- et -B- sont tétravalents, -NR- et -NR'- sont trivalents et -X, -Y et -Z sont bivalents.

Le composé de l'invention sous la forme réduite ne présente pas de ferromagnétisme; sous la forme oxydée, des propriétés ferromagnétiques sont nettement observables. Les radicaux -L et -L' sont le motif de base à l'origine du ferromagnétisme. A cause de la structure du radical -B-, une interaction entre le substituant Y qu'il porte et les radicaux -NR- et -NR'- qui lui sont liés est impossible. Une interaction acido-basique pourra par contre se former entre le substituant Y acide et l'un des radicaux -NR- et -NR'-situés du côté opposé aux motifs -A- ou -A'- les plus proches, permettant ainsi l'apparition du ferromagnétisme. Si les radicaux -X et -Z sont basiques, alors que Y est un site acide, ou si les radicaux -X et -Z sont acides alors que Y est un site basique, l'interaction peut également conduire à la formation d'un sel interne et contribuer au ferromagnétisme du composé moléculaire.

Les radicaux -A-Z et -A'- ont pour fonction, du fait de leur encombrement stérique, d'orienter la stéréochimie des enchaînement lors de la synthèse et de bloquer la rotation autour des liaisons B-N dans le composé moléculaire.

Selon une première forme d'exécution de la présente invention, -A- est un radical issu d'un noyau benzénique multivalent en position para portant un à quatre substituants Rₐ choisis indépendamment parmi un radical hydro, un radical alkyle, un radical cycloalkyle, un radical alcoxyle, un radical alcoxyalkyle, un radical nitrile, un radical nitro et un radical hydroxyalkyle, de la forme:

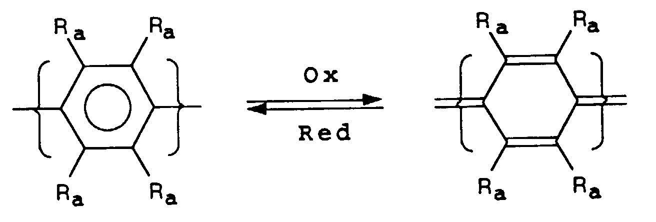

Selon une deuxième forme d'exécution, -A- est un radical multivalent issu d'un noyau naphtalène portant un à quatre substituants Rb en positions 5, 6, 7 et/ou 8 choisis indépendamment parmi un radical hydro, un radical alkyle, un radical cycloalkyle, un radical alcoxyle, un radical alcoxyalkyle, un radical nitrile, un radical nitro, un radical hydroxyalkyle, un radical fluoro, un radical chloro, un radical bromo et un radical iodo, et portant un à deux substituants R_{c} en position 2 et/ou 3 choisis indépendamment parmi un radical hydro, un radical alkyle, un radical cycloalkyle, un radical alcoxyle et un radical alcoxyalkyle, de la forme:

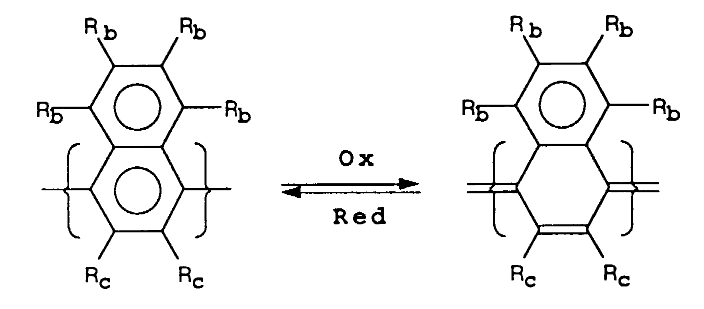

Selon une troisième forme d'exécution, -A- est un radical multivalent issu d'un noyau tétrahydronaphtalène portant un à quatre substituants R_{d} en positions 5, 6, 7 et/ou 8 choisis indépendamment parmi un radical hydro, un radical alkyle, un radical cycloalkyle, un radical alcoxyle, un radical alcoxyalkyle, un radical hydroxyalkyle, un radical fluoro, un radical chloro, un radical bromo et un radical iodo, et portant un à deux substituants Rₑ en position 2 et/ou 3 choisis parmi un radical hydro, un radical alkyle, un radical cycloalkyle, un radical alcoxyle et un radical alcoxyalkyle, de la forme:

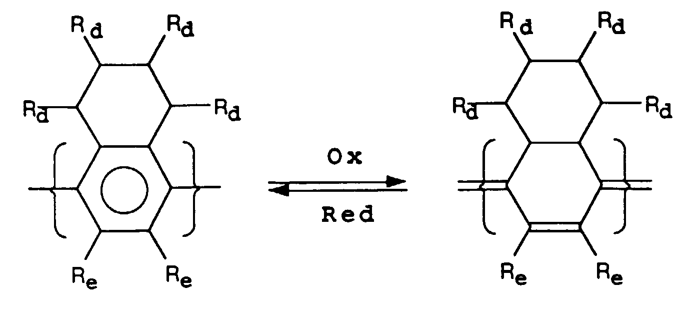

Selon une quatrième forme d'exécution, -A'- est un radical issu d'un noyau benzènique multivalent en position para portant un à quatre substituants choisis indépendamment parmi un radical hydro, un radical alkyle, un radical cycloalkyle, un radical alcoxyle, un radical alcoxyalkyle, un radical nitrile, un radical nitro et un radical hydroxyalkyle.

Selon une cinquième forme d'exécution, -A-' est un radical issu d'un noyau benzénique multivalent en position ortho portant un à quatre substituants R_{f} choisis indépendamment parmi un radical hydro, un radical alkyle, un radical cycloalkyle, un radical alcoxyle, un radical alcoxyalkyle et un radical hydroxyalkyle.

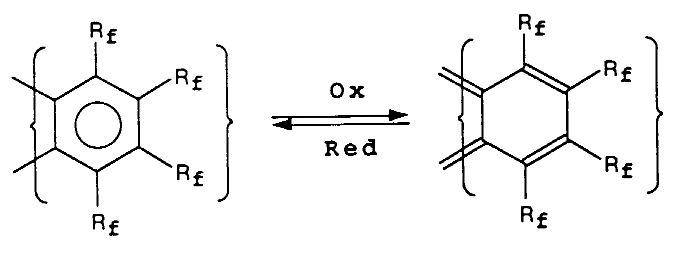

Selon une sixième forme d'exécution, -B- est un radical multivalent issu d'un noyau naphtalène portant un à deux substituants R_{g} en position 2 et/ou 3 sur le noyau multivalent choisis indépendamment parmi un radical hydro, un radical nitrile, un radical nitro, un radical fluoro, un radical chloro, un radical bromo et un radical iodo, et portant sur l'autre noyau ledit substituant Y en position 6 ou 7, et un à trois substituants Rₕ en position 5, 8, et/ou en position disponible 6 ou 7 choisis indépendamment parmi un radical hydro, un radical alkyle, un radical cycloalkyle, un radical alcoxyle, un radical alcoxyalkyle, un radical nitrile, un radical nitro, un radical hydroxyalkyle, un radical fluoro, un radical chloro, un radical bromo et un radical iodo, de la forme:

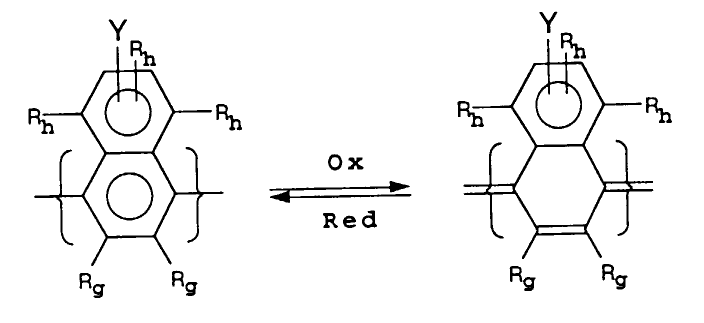

Selon une septième forme d'exécution, -B- est un radical multivalent issu d'un noyau benzénique portant ledit substituant Y relié au noyau par un maillon paraphénylène en position 2 ou 3, ledit maillon portant ledit substituant Y en position 4 et un à deux substituants Rᵢ en position 2 et/ou 6 choisis indépendamment parmi un radical hydro, un radical alkyle, un radical cycloalkyle, un radical alcoxyle, un radical alcoxyalkyle et un radical hydroxyalkyle, de la forme:

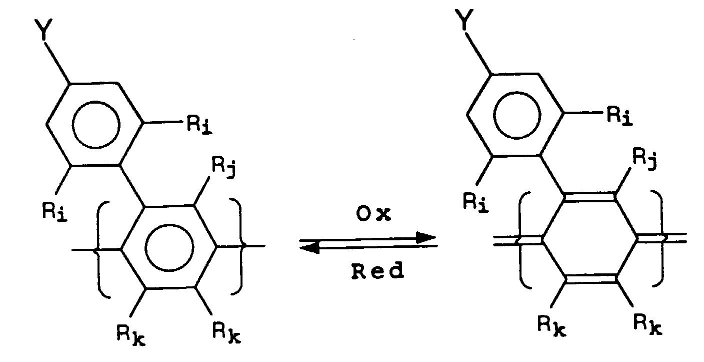

Dans ce cas, le radical -B- peut être considéré comme dérivant du biphényle. Le maillon paraphénylène constitue un bras rigide reliant le radical multivalent par le carbone numéro 1 au substituant Y en position para par le carbone numéro 4. Les positions 2 et 6 sont libres de recevoir un ou deux susbstituants Rᵢ.

Selon une huitième forme d'exécution, -B- est un radical multivalent issu d'un noyau benzénique portant ledit premier substituant Y relié au noyau par un maillon éthynylène ou un maillon paraphénylène éventuellement substitué, ledit maillon étant en position 2 ou 3, un deuxième substituant Rⱼ sur l'autre position 2 ou 3 choisi indépendamment parmi un radical hydro, un radical alkyle, un radical cycloalkyle, un radical alcoxyle, un radical alcoxyalkyle, un radical hydroxyalkyle, un radical fluoro, un radical chloro, un radical bromo et un radical iodo, et un troisième et/ou quatrième substituant Rₖ en position 5 et/ou 6 choisi indépendamment parmi un radical hydro, un radical fluoro, un radical chloro, un radical bromo et un radical iodo.

Si Le radical -B- porte un maillon éthynylène, il peut être considéré comme dérivant du phénylacétylène. Il est de la forme:

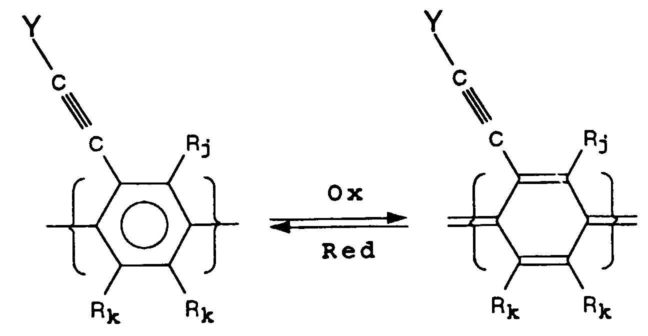

Selon une neuvième forme d'exécution, -R et -R' sont indépendamment choisis parmi un radical hydro, un radical alkyle, un radical cycloalkyle et un radical hydroxyalkyle.

Dans l'état réduit, les radicaux -NR- et -NR'- sont bivalents et relient un radical -A- réduit à un radical -B-réduit; dans l'état oxydé, ces radicaux sont trivalents et relient un radical -A- oxydé à un radical -B- réduit ou bien un radical -A- réduit à un radical -B- oxydé.

La présente invention a également pour objet un procédé de fabrication du composé selon l'invention comprenant l'enchaînement d'un moins un composé aminoaromatique, éventuellement substitué, avec un acide aminosulfonique à deux noyaux benzèniques condensés, ledit enchaînement étant réalisée en solution alcoolique, en présence d'un acide faible et d'un agent oxydant.

De préférence l'agent oxydant est choisi parmi le peroxyde d'hydrogène et le peroxodisulfate d'ammonium.

De préférence, ledit acide aminosulfonique à deux noyaux benzéniques condensés est choisi parmi les acides de CLEVE comme l'acide 5-amino-2-napthtalènesulfonique (forme β) et l'acide 8-amino-2-naphtalènesulfonique (forme θ).

De préférence, ledit composé aminoaromatique est choisi parmi une paraphénylènediamine, une N,N-dialkylparaphénylènediamine et une N,N-dialkylaniline.

Selon une variante de mise en oeuvre, le procédé comporte en outre la réduction par un réducteur fort et l'enchaînement avec un composé aminoaromatique substitué.

Selon une première forme d'exécution de l'invention, ledit composé aromatique substitué porte un premier substituant halogène et un second substituant choisi parmi un substituant cyano en position para par rapport à l'halogène, un substituant nitro en position para par rapport à l'halogène, et deux substituants nitro en positions ortho et para par rapport à l'halogène.

Selon une deuxième forme d'exécution de l'invention, ledit composé aromatique substitué est une arylamine dialkylée sur l'azote.

De préférence, ledit réducteur fort est l'hydrazine en présence de nickel Raney.

Selon une autre variante de mise en oeuvre, le procédé de fabrication comporte en outre une étape de purification et éventuellement une étape de cristallisation dudit composé selon l'invention.

Selon encore une autre variante de mise en oeuvre, le procédé de fabrication comporte en outre une étape d'oxydation dudit composé selon l'invention dans laquelle l'oxydation est effectuée par un peroxodisulfate en solution aqueuse. Les produits de condensation à l'état réduit ne présentent pas de ferromagnétisme à la température ambiante. On obtient un composé de ferromagnétique en le soumettant à une oxydation. Celle-ci s'effectue de préférence par un peroxodisulfate en solution aqueuse.

La synthèse du composé de l'invention s'effectue ainsi à partir de sous-ensembles comportant plusieurs radicaux déjà liés.

Bien que des composés moléculaires comportant plus de deux radicaux -L soient réalisables par un procédé analogue, leur intérêt est limité. En effet d'une part le nombre d'étapes du procédé nécessaire à leur réalisation augmente rapidement, d'autre part le rendement de la réaction chute de manière importante au profits d'espèces secondaires non ferromagnétiques.

D'autres caractéristiques et avantages de la présente invention apparaîtront à la lecture des exemples suivants de réalisation, donnés bien entendu à titre illustratif mais nullement limitatif, en référence au dessin annexé.

Dans le dessin annexé:
- la figure 1 montre le spectre de résonance ferromagnétique du composé selon la présente invention à l'état partiellement oxydé,
- la figure 2 montre le spectre de résonance ferromagnétique du composé selon l'invention après oxydation.

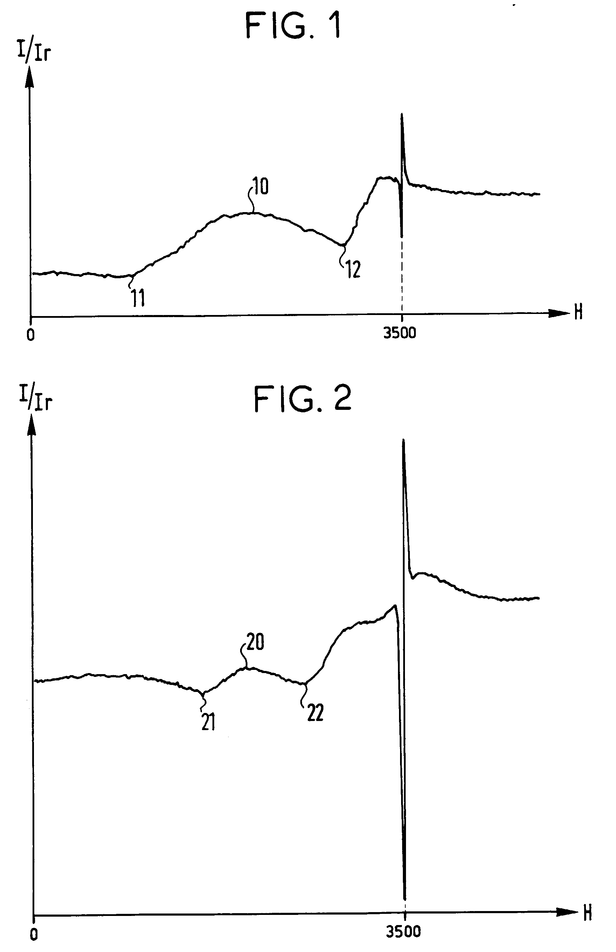

Sur les figures 1 et 2, le champ magnétique H en 10⁻⁴ Tesla (1 Gauss) est donné en abscisse et en ordonnée, graduée en unités arbitraires relatives, est donné le rapport I/Iᵣ entre l'absorption du composé et une absorption de référence.

### EXEMPLE 1

Dans un erlenmeyer contenant 40ml d'éthanol et 5ml d'acide acétique, on introduit 1,36g de N,N-diméthylparaphénylènediamine (10⁻² mole) et 2,23g d'acide 5-amino-2-naphtalènesulfonique (forme β de l'acide de CLEVE) en poudre fine. On y ajoute 1,2ml d'une solution à 50% en poids de peroxyde d'hydrogène (2.10⁻² mole). Le mélange est maintenu sous agitation à température ambiante pendant 30mn, puis la suspension est filtrée. Le filtrat est versé dans 200ml d"eau. On observe l'apparition d'un précipité bleu-vert que l'on récupère par centrifugation. Le produit est ensuite lavé avec du N-propanol puis séché.

Ce produit ne présente pas de caractère ferromagnétique à température ambiante, ni à 119°K. Il est obtenu selon la réaction:

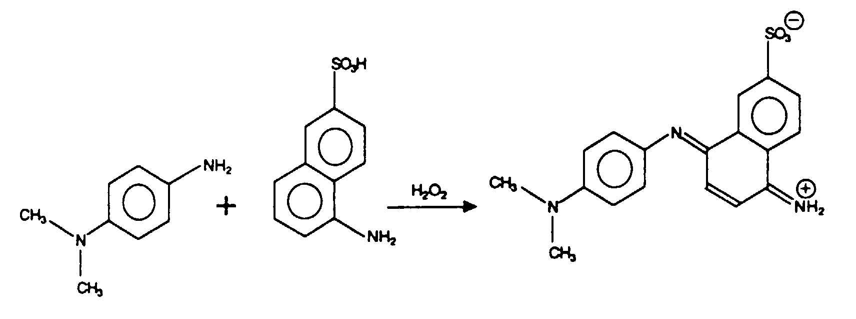

Le produit précédemment obtenu est dispersé dans 30ml d'éthanol contenant 1g de nickel de Raney et 5ml d'hydrazine anhydre. L'ensemble est lentement porté à ébullition en 30mn et maintenu à reflux pendant 15mn à cette température. La totalité du nickel de Raney est extraite par un aimant. Après filtration et évaporation du solvant, on récupère un produit réduit ne présentant pas de caractère ferromagnétique à température ambiante, qui a pour formule:

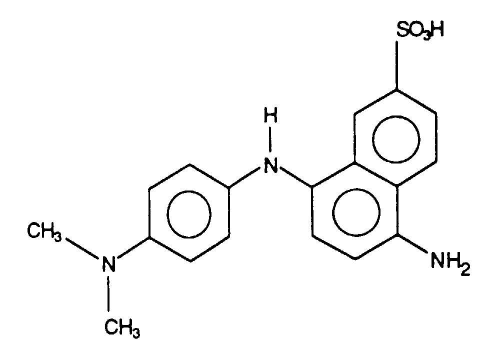

Le résidu est chauffé à reflux à 140°C pendant 4 heures dans 20ml de N-butanol en présence de 2g de 1-chlore-2,4-dinitrobenzène. On obtient le composé selon la réaction:

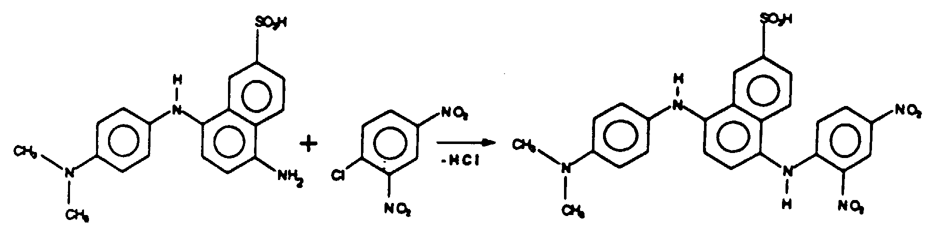

La suspension dans le N-butanol est refroidie puis diluée dans 50ml d'acétone. Le tout est versé dans 300ml d'eau additionnée de 2,25g de peroxodisulfate d'ammonium. Après 30mn d'oxydation à température ambiante et sous agitation le précipité est récupéré par centrifugation, lavé à l'eau, puis à l'acétone et à l'éther éthylique, et enfin séché. On obtient avec un rendement de 4%, 226mg d'une poudre bleu foncé à caractère ferromagnétique. Le composé oxydé présente alors une structure iminoquinonique comprise entre les formes limites suivantes:

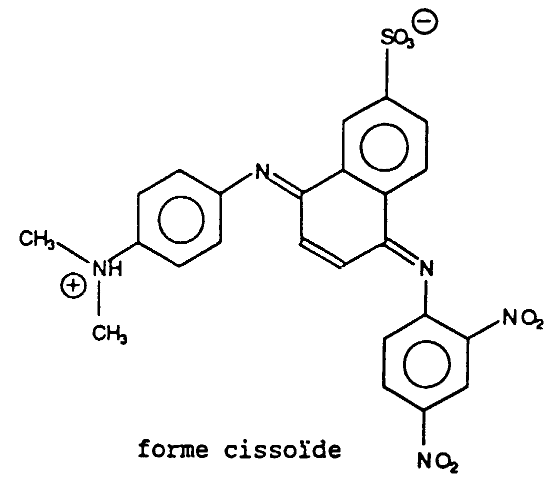

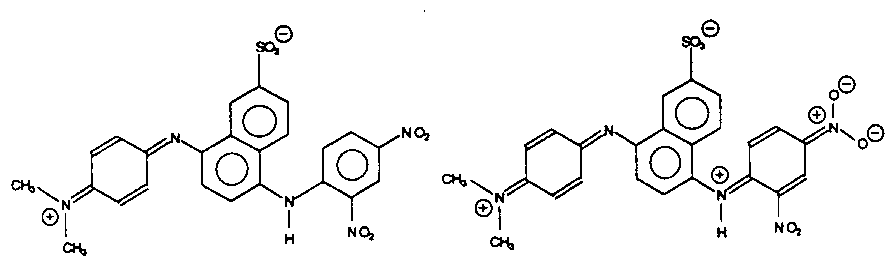

Le composé de forme cissoïde, après lavage et séchage, se réarrange par formation d'un sel interne entre XA- et -BY-, avec pour conséquence la création d'un biradical à l'état triplet ainsi que l'aromatisation du radical substitué -BY-. Le composé selon l'invention se présente alors sous la forme suivante:

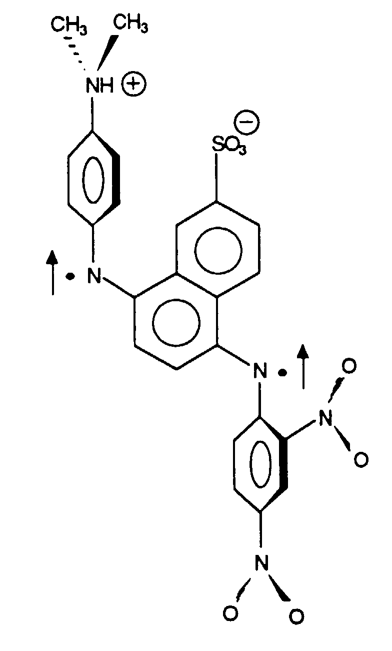

Des spectres de résonnance ferromagnétique ont été réalisés à température ambiante à la fréquence de 9,43GHz. Les courbes obtenues sont les courbes dérivées de résonnance.

Le composé selon l'invention, à l'état partiellement oxydé par un séjour de 30mn dans une solution aqueuse de peroxodisulfate d'ammonium, présente deux bandes de résonnance ferromagnétiques que l'on peut voir sur le spectre de résonance ferromagnétique de la figure 1 du dessin (courbe 10): un premier pic 11 correspondant à un champ de 3130.10⁻⁴ Tesla (3130 Gauss) et un second pic 12 correspondant à un champ de 1560.10⁻⁴ Tesla (1560 Gauss).

Après oxydation prolongée par un séjour de 48h dans la même solution trois fois plus concentrée, le composé selon l'invention présente un spectre de résonnance plus complexe. Sur la figure 2 du dessin (courbe 20), une première bande 21 correspondant à un champ de 3450.10⁻⁴ Tesla (3450 Gauss), une deuxième bande 22 correspondant à un champ de 2680.10⁻⁴ Tesla (2680 Gauss) et une troisième bande correspondant à un champ de 1820.10⁻⁴ Tesla (1820 Gauss) sont visibles. La réaction d'oxydation étant relativement lente, on peut d'ailleurs observer une augmentation progressive du ferromagnétisme au fur et à mesure qu'elle se poursuit.

### EXEMPLE 2

Dans un bêcher contenant 25ml d'acide acétique, on dissout 1,21g de N,N-diméthylaniline (10⁻² mole), et 1,36g de N,N-diméthyl-paraphénylènediamine (10⁻² mole). Une solution constituée de 2,23g d'acide de CLEVE β (10⁻² mole), 2ml d'ammoniaque (d=0,89) et 25ml d'eau est ajoutée dans le bêcher. On introduit alors 1,75ml d'une solution à 50% en poids de peroxyde d'hydrogène (3.10⁻² mole). La réaction a lieu à température ambiante pendant une heure sous agitation. La filtration permet d'éliminer l'acide de CLEVE n'ayant pas réagi. Le filtrat est dilué dans 200ml d'eau et le pH de la suspension est amené à 7 par ajout d'ammoniaque concentrée. La suspension est centrifugée , puis le culot est lavé par des opérations successives de redispersion dans un solvant et de centrifugation. L'opération est renouvelée trois fois avec de l'eau, trois fois avec de l'éthanol et enfin une fois avec du diéthyloxyde.

Après séchage, on obtient avec un rendement de 30% une poudre de couleur bleu-vert contenant le produit semi-oxydé suivant, mêlé à d'autres produits secondaires:

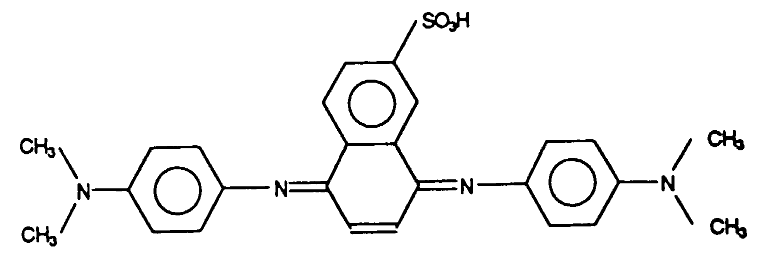

Cette poudre présente une aimantation de l'ordre de 0,05emu/g.

### EXEMPLE 3

Dans un bêcher contenant 25ml d'acide acétique, on dissout 2,42g de N,N-diméthylaniline (2.10⁻² mole), et 1,08g de paraphénylènediamine (10⁻² mole). Une solution constituée de 4,46g d'acide de CLEVE θ (2.10⁻² mole), 4ml d'ammoniaque (d=0,89) et 50ml d'eau est ajoutée dans le bêcher. on introduit alors 13,7g de peroxodisulfate d'ammonium par pincées durant une heure. La réaction a lieu à température ambiante pendant une heure sous agitation.

On procède de la même manière que dans l'exemple 2 pour la filtration et la centrifugation.

Après séchage, on obtient avec un rendement de 20% une poudre de couleur bleu-vert contenant le produit semi-oxydé suivant, mêlé à d'autres produits secondaires:

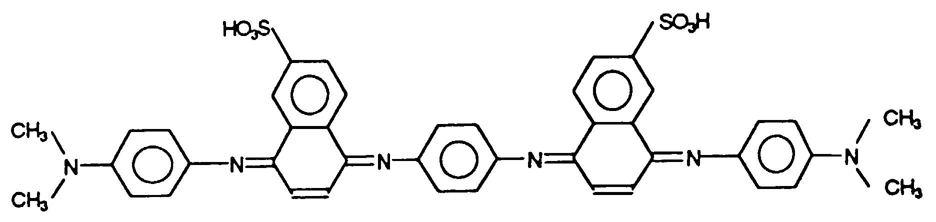

Cette poudre présente une aimantation de l'ordre de 0,03emu/g.

Bien entendu la présente invention n'est pas limitée au mode de réalisation décrit, mais elle est susceptible de nombreuses variantes accessibles à l'homme de l'art sans que l'on ne s'écarte de l'esprit de l'invention.

## Revendications

1. Composé organique possédant des propriétés ferromagnétiques, caractérisé en ce qu'il est un composé moléculaire répondant à l'une des formules générales suivantes:
(I) L-A-Z
(II) L-A'-L'
dans lesquelles:
. -A- est choisi parmi un radical issu d'un noyau benzènique multivalent en position para et éventuellement substitué, un radical issu d'un noyau naphtalène multivalent en position para de l'un des noyaux et éventuellement substitué,
. -Z est un radical mono ou bivalent choisi parmi un radical hydro, un radical nitrile, un radical nitro, un radical fluoro, un radical chloro, un radical bromo, un radical iodo, un radical amino, un radical imino, un radical alkylamino, un radical dialkylamino, un radical dialkyliminio, un radical trialkylammonio, un radical aminocyclique dont un atome de carbone du cycle peut-être remplacé par un hétéroatome, un radical sulfo, un radical carboxy, un radical phosphono, un radical arsono, un radical acyle, un radical formamido, un radical acétamido et un radical acétimido.
. -A'- est choisi parmi un radical issu d'un noyau benzènique multivalent en position para ou ortho et éventuellement substitué,
. -L et -L' sont de la forme: 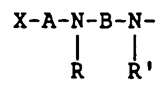 N étant l'atome d'azote, où:
. -X est un radical mono ou bivalent susceptible de fixer un proton, choisi parmi un premier groupe de composés constitué d'un radical amino, un radical imino, un radical alkylamino, un radical dialkylamino, un radical dialkyliminio, un radical trialkylammonio et un radical aminocyclique dont un atome de carbone du cycle peut-être remplacé par un hétéroatome, et un deuxième groupe de composés constitué d'un radical sulfo, un radical carboxy, un radical phosphono et un radical arsono,
. -NR- et -NR'- sont des radicaux multivalents issus d'une amine primaire ou de l'ammoniac,
. -B- est choisi parmi un radical issu d'un noyau naphtalène multivalent en position para de l'un des noyaux et portant au moins un substituant Y en position 6 ou 7 sur l'autre noyau, un radical issu d'un noyau benzènique multivalent et portant au moins un substituant Y en position 2 ou 3 relié au noyau par un maillon éthynylène ou un maillon paraphénylène éventuellement substitué, ledit substituant Y étant choisi parmi un radical sulfo, un radical carboxy, un radical phosphono et un radical arsono si -X est choisi dans ledit premier groupe de composés, et ledit substituant Y étant choisi parmi un radical amino, un radical imino, un radical alkylamino, un radical dialkylamino, un radical dialkyliminio, un radical trialkylammonio, un radical aminocyclique dont un atome de carbone du cycle peut-être remplacé par un hétéroatome si -X est choisi dans ledit deuxième groupe de composés, ledit composé organique étant sous sa forme réduite ou sous sa forme oxydée.

2. Composé selon la revendication 1, dans lequel -A- est un radical issu d'un noyau benzènique en position para portant un à quatre substituants choisis indépendamment parmi un radical hydro, un radical alkyle, un radical cycloalkyle, un radical alcoxyle, un radical alcoxyalkyle, un radical nitrile, un radical nitro et un radical hydroxyalkyle.

3. Composé selon la revendication 1, dans lequel -A- est un radical multivalent issu d'un noyau naphtalène portant un à quatre substituants en positions 5, 6, 7 et/ou 8 choisis indépendamment parmi un radical hydro, un radical alkyle, un radical cycloalkyle, un radical alcoxyle, un radical alcoxyalkyle, un radical nitrile, un radical nitro, un radical hydroxyalkyle, un radical fluoro, un radical chloro, un radical bromo et un radical iodo, et portant un à deux substituants en position 2 et/ou 3 choisis indépendamment parmi un radical hydro, un radical alkyle, un radical cycloalkyle, un radical alcoxyle et un radical alcoxyalkyle.

4. Composé selon la revendication 1, dans lequel -A- est un radical multivalent issu d'un noyau tétrahydronaphtalène portant un à quatre substituants en positions 5, 6, 7 et/ou 8 choisis indépendamment parmi un radical hydro, un radical alkyle, un radical cycloalkyle, un radical alcoxyle, un radical alcoxyalkyle, un radical hydroxyalkyle, un radical fluoro, un radical chloro, un radical bromo et un radical iodo, et portant un à deux substituants en position 2 et/ou 3 choisis indépendamment parmi un radical hydro, un radical alkyle, un radical cycloalkyle, un radical alcoxyle et un radical alcoxyalkyle.

5. Composé selon la revendication 1, dans lequel -A'- est un radical issu d'un noyau benzènique multivalent en position para portant un à quatre substituants choisis indépendamment parmi un radical hydro, un radical alkyle, un radical cycloalkyle, un radical alcoxyle, un radical alcoxyalkyle, un radical nitrile, un radical nitro et un radical hydroxyalkyle.

6. Composé selon la revendication 1, dans lequel -A'- est un radical issu d'un noyau benzènique multivalent en position ortho portant un à quatre substituants choisis indépendamment parmi un radical hydro, un radical alkyle, un radical cycloalkyle, un radical alcoxyle, un radical alcoxyalkyle et un radical hydroxyalkyle.

7. Composé selon la revendication 1, dans lequel -B- est un radical multivalent issu d'un noyau naphtalène portant un à deux substituants, en position 2 et/ou 3 sur le noyau multivalent choisis indépendamment parmi un radical hydro, un radical nitrile, un radical nitro, un radical fluoro, un radical chloro, un radical bromo et un radical iodo, et portant sur l'autre noyau ledit substituant Y en position 6 ou 7, et un à trois substituants, en position 5, 8, et/ou en position disponible 6 ou 7, choisis indépendamment parmi un radical hydro, un radical alkyle, un radical cycloalkyle, un radical alcoxyle, un radical alcoxyalkyle, un radical nitrile, un radical nitro, un radical hydroxyalkyle, un radical fluoro, un radical chloro, un radical bromo et un radical iodo.

8. Composé selon la revendication 1, dans lequel -B- est un radical multivalent issu d'un noyau benzènique portant ledit substituant Y relié au noyau par un maillon paraphénylène en position 2 ou 3, ledit maillon portant ledit substituant Y en position 4 et un à deux substituants en position 2 ou 6 choisis indépendamment parmi un radical hydro, un radical alkyle, un radical cycloalkyle, un radical alcoxyle, un radical alcoxyalkyle et un radical hydroxyalkyle.

9. Composé selon la revendication 1, dans lequel -B- est un radical multivalent issu d'un noyau benzènique portant ledit premier substituant Y relié au noyau par un maillon éthynylène ou un maillon paraphénylène éventuellement substitué, ledit maillon étant en position 2 ou 3, un deuxième substituant sur l'autre position 2 ou 3 choisi indépendamment parmi un radical hydro, un radical alkyle, un radical cycloalkyle, un radical alcoxyle, un radical alcoxyalkyle, un radical hydroxyalkyle, un radical fluoro, un radical chloro, un radical bromo et un radical iodo, et un troisième et/ou quatrième substituant en position 5 et/ou 6 choisi indépendamment parmi un radical hydro, un radical fluoro, un radical chloro, un radical bromo et un radical iodo.

10. Composé selon la revendication 1, dans lequel -R et -R' sont choisis indépendamment parmi un radical hydro, un radical alkyle, un radical cycloalkyle et un radical hydroxyalkyle.

11. Procédé de fabrication du composé selon l'une des revendications précédentes, comprenant l'enchaînement d'au moins un composé aminoaromatique, éventuellement substitué, avec un acide aminosulfonique à deux noyaux benzèniques condensés, ledit enchaînement étant réalisé en solution alcoolique, en présence d'un acide faible et d'un agent oxydant.

12. Procédé selon la revendication 11, dans lequel ledit agent oxydant est choisi parmi le peroxyde d'hydrogène et le peroxodisulfate d'ammonium.

13. Procédé selon l'une des revendications 11 à 12, dans lequel ledit acide aminosulfonique à deux noyaux benzèniques condensés est choisi parmi l'acide 8-amino-2-naphtalènesulfonique, et l'acide 5-amino-2-naphtalènesulfonique.

14. Procédé selon l'une des revendications 11 à 13, dans lequel ledit composé aminoaromatique est choisi parmi une paraphénylènediamine, une N,N-dialkylparaphénylènediamine et une N,N-dialkylaniline.

15. Procédé selon l'une des revendications 11 à 14, comportant en outre la réduction par un réducteur fort et la condensation avec un composé aminoaromatique substitué.

16. Procédé selon la revendication 15, dans lequel ledit composé aromatique substitué porte un premier substituant halogène et un second substituant choisi parmi un substituant cyano en position para par rapport à l'halogène, un substituant nitro en position para par rapport à l'halogène, et deux substituants nitro en positions ortho et para par rapport à l'halogène.

17. Procédé selon la revendication 15, dans lequel ledit composé aromatique substitué est une arylamine dialkylée sur l'azote.

18. Procédé selon l'une des revendications 15 à 17, dans lequel ledit réducteur fort est l'hydrazine en présence de nickel Raney.

19. Procédé de fabrication selon l'une des revendications 11 à 18, comportant en outre une étape de purification dudit composé.

20. Procédé de fabrication selon la revendication 19, comportant en outre une étape de cristallisation dudit composé.

21. Procédé de fabrication selon l'une des revendications 11 à 20, comportant en outre une étape d'oxydation dudit composé.

22. Procédé de fabrication selon la revendication 21, dans lequel ladite oxydation est effectuée par un peroxodisulfate en solution aqueuse.
